# EUROPÄISCHE PATENTSCHRIFT

(11) **EP 3 241 577 B1**
(45) Veröffentlichungstag und Bekanntmachung des Hinweises auf die Patenterteilung: **18.11.2020**
(21) Anmeldenummer: 17172400.8
(22) Anmeldetag: 27.04.2007
(51) Int. Cl.: A61M 1/36, A61M 1/34

(54) **VERFAHREN ZUR FÜLLUNG UND SPÜLUNG EINES BLUTSCHLAUCHSATZES**
METHOD FOR FILLING AND RINSING A SET OF BLOOD LINES
PROCÉDÉ DE REMPLISSAGE ET DE RINÇAGE D'UN JEU DE TUYAU DE SANG

(30) Priorität: 11.05.2006 DE 102006022122
(43) Veröffentlichungstag der Anmeldung: 08.11.2017
(62) Teilanmeldung aus: 07724694.0
(73) Patentinhaber: Fresenius Medical Care Deutschland GmbH, 61352 Bad Homburg (DE)
(72) Erfinder: Fischer, Max, 60323 Frankfurt (DE)
(74) Vertreter: Behr, Wolfgang

(56) Entgegenhaltungen:
- WO-A-96/40320
- WO-A-99/20376
- DE-A1- 4 240 681
- DE-C1- 10 011 208
- US-A- 5 776 345

## Beschreibung

Die Erfindung betrifft ein Verfahren zur Füllung und Spülung eines Blutschlauchsatzes, der ein Pumpsegment für eine Blutpumpe, eine mit einem Eingang eines Dialysators verbundene arterielle Leitung und eine mit einem Ausgang eines Dialysators verbundene venöse Leitung umfasst, über eine Substituatleitung.

Solche Blutschlauchsätze kommen bei extrakorporalen Bluttherapien, z.B. bei der Hämodialyse, zum Einsatz und bilden dabei den extrakorporalen Blutkreislauf. Als arterielle und venöse Leitung eines solchen Blutschlauchsatzes werden üblicherweise steril verpackte Einmalartikel verwendet, welche vor der Behandlung gefüllt und gespült werden müssen. Dieses Füllen und Spülen des Blutschlauchsatzes wird auch Priming genannt und dient zur Vermeidung des Kontaktes des Blutes mit Luft bei der extrakorporalen Therapie. Hierbei wird das als Disposable-System ausgeführte Blutschlauchsystem mit Substituat gefüllt, bevor der Patient zur Behandlung angeschlossen wird, so dass das extrakorporale System nach dem Priming nahezu luftfrei ist. Nach dem Füllen und Spülen des Schlauchsystems werden venöse und arterielle Leitungen üblicherweise miteinander verbunden, so dass das Substituat in diesem Kreislauf aus arterieller Leitung, Dialysator und venöser Leitung zirkulieren kann, bis der Patient an das System angeschlossen wird. Nach dem Anschluss des Patienten wird das Substituat durch das einströmende Blut verdrängt, so dass ein möglicher Kontakt des Blutes mit der Luft auf ein Minimum reduziert wird.

Verfahren zur Füllung und Spülung von Blutschlauchsätzen unter Verwendung von Beuteln mit physiologischer Lösung und entsprechenden Beuteln zum Auffangen der benutzten Lösung sind bekannt. Dabei wird üblicherweise ein Beutel mit Kochsalzlösung mit der arteriellen Leitung verbunden und diese gefüllt. Nach dem Füllen der arteriellen Leitung wird nun die Blutpumpe verwendet, um auch die venöse Leitung zu füllen und zu spülen. Hierzu wird das Pumpsegment des Blutschlauchsatzes, ein Schlauchabschnitt mit bestimmten Eigenschaften, in die Blutpumpe, üblicherweise eine peristaltische Pumpe bzw. eine Rollenpumpe, eingesetzt, so dass die Blutpumpe über das Pumpsegment Kochsalzlösung in die venöse Leitung pumpen kann.

Um den Füll- und Spülvorgang weitestgehend zu automatisieren, wurde in EP 831 945 B1 ein Verfahren vorgeschlagen, bei welchem ein T-Konnektor zum Einsatz kommt, um zum Füllen und Spülen die arterielle Leitung und die venöse Leitung kurzzuschließen und über den T-Konnektor mit einem Beutel zum Auffangen der benutzten Lösung zu verbinden. Des weiteren sind an den Blutleitungen, das heißt der arteriellen und der venösen Leitung, in der Nähe des Konnektors zwingend Ventile vorgesehen, um den Füll- und Spülvorgang zu steuern. Zuerst wird hierbei das Substituat durch die Verwendung der Schwerkraft von einem Beutel in die arterielle Leitung gefüllt. Nach Öffnen und Schließen der entsprechenden Ventile wird daraufhin die Flüssigkeit von der arteriellen Leitung in die venöse Leitung gefüllt. Nach einem weiteren Öffnen und Schließen der entsprechenden Ventile kann dann das Substituat in dem Kreislauf aus arterieller Leitung, Dialysator und venöser Leitung durch die Blutpumpe zirkuliert werden. Das Anbringen der ansteuerbaren Ventile am Blutschlauchsatz ist dabei umständlich und darf nicht vergessen werden, erfordert zusätzlich elektrische und pneumatische Verbindungen und ist anfällig für Bedienungsfehler der Dialyseschwester, z. B. durch Verwechslung oder fehlerhafte Positionierung der Ventile. Zudem ist es nötig, vor Beginn der automatisierbaren Prozedur manuell eine Quelle mit einem sterilen Fluid (z. B. ein Beutel mit steriler Kochsalzlösung) oberhalb der Behandlungsmaschine aufzuhängen, anzustechen und mit dem Blutschlauchsatz zu verbinden, wobei sichergestellt werden muss, dass die Ventile zuvor richtig montiert und geschlossen sind. Zur Automatisierung der Ventilbetätigung müssen die Ventile zudem hard- und softwareseitig angesteuert werden, wobei gleichzeitig sichergestellt werden muss, dass keines der Ventile versagt oder sich beim Anschließen des Lösungsbeutels in einer falschen Verschlussstellung befindet.

WO 99/20367 zeigt ein Verfahren zum Füllen und Spülen eines Blutschlauchsatzes, wobei die Priminglösung stromabwärts der Blutpumpe in die arterielle Leitung zugegeben wird und gravimetrisch entgegen der normalen Richtung des Blutflusses durch diese hindurchfließt, während gleichzeitig durch Betrieb der Blutpumpe Flüssigkeit durch den Dialysator und die venöse Leitung gepumpt wird, wobei die Enden der arteriellen und der venösen Leitung miteinander in Verbindung stehen

DE 42 40 681 A1 zeigt eine Blutbehandlungsvorrichtung, bei welcher ein Behälter mit Spülflüssigkeit stromauf der Blutpumpe über eine Verbindungsleitung an den Blutweg angeschlossen ist, ein okkludierender Aktor an der Verbindungsleitung angeordnet ist und eine Steuereinheit zur Betätigung des Aktors vorgesehen ist, die in vorbestimmten Zeitabständen die Vorrichtung in eine erste Phase (Spülphase) dadurch schaltet, dass die Klemmwirkung des Aktors aufgehoben wird, so dass durch die Wirkung der Blutpumpe eine vorbestimmte Spülflüssigkeitsmenge durch den Blutweg geführt wird, und anschließend in eine zweite Phase (Dialysebehandlung) schaltet, bei der die Ultrafiltrationseinheit so betrieben wird, dass zusätzlich zu der dem Patienten zu entziehenden Blutwassermenge die in der ersten Phase zugeführte Spülflüssigkeitsmenge dem Patienten wieder entzogen wird. Als okkludierender Aktor kann eine peristaltische Pumpe eingesetzt werden.

US 5 776 345 A zeigt eine Blutbehandlungsvorrichtung mit einer Substituatleitung, in welcher eine Substituatpumpe angeordnet ist.

Aufgabe der vorliegenden Erfindung ist es, ein Verfahren zur Füllung und Spülung eines Blutschlauchsatzes zur Verfügung zu stellen, welches sicherer in der Anwendung und einfacher, schneller und kostengünstiger durchführbar ist.

Erfindungsgemäß wird die Aufgabe von einem Verfahren zur Füllung und Spülung eines Blutschlauchsatzes gemäß Anspruch 1 erfüllt. Es umfasst dabei die an sich bekannten Schritte des Verbindens der arteriellen Leitung mit der venösen Leitung sowie des Verbindens dieser beiden Leitungen mit einem Rinse-Port, Verbinden der Substituatleitung mit einem Substituat-Port, Öffnen des Rinse-Ports, Füllen der arteriellen und der venösen Leitung, Spülen der arteriellen und der venösen Leitung, Schließen des Rinse-Ports, Zirkulieren des Substituats im Kreislauf aus arterieller Leitung, Dialysator und venöser Leitung durch die Blutpumpe, wobei die Blutpumpe in der arteriellen Leitung angeordnet ist und das Füllen und/oder das Spülen der arteriellen und der venösen Leitung gleichzeitig erfolgt, wobei Substituat aus der Substituatleitung zugeführt wird. Das Verfahren ist dadurch gekennzeichnet, dass über eine Substituatpumpe Substituat zwischen Blutpumpe und Dialysator zugeführt wird. Natürlich wird vor Verwendung der Blutpumpe das Pumpsegment des Blutschlauchsatzes in diese eingesetzt.

Insbesondere kann durch dieses Verfahren darauf verzichtet werden, Ventile am Blutschlauchsatz anzubringen, welche das Füllen oder Spülen der arteriellen Leitung und das darauf folgende Füllen oder Spülen der venösen Leitung steuern müssten. Die mit dem Anbringen dieser Ventile verbundenen Fehlerquellen bei der Bedienung sind damit ausgeschlossen. Zudem ermöglicht das gleichzeitige Füllen und/oder Spülen der arteriellen und der venösen Leitungen einen schnelleren Füll-und Spülvorgang, da auf unterschiedliche Verfahrensschritte verzichtet werden kann. Zudem ist das Verfahren auch einfacher zu automatisieren und die Zeit, welche eine Dialyseschwester zur Füllung und Spülung des Blutschlauchsatzes benötigt, wird minimiert. Außerdem ist so der Blutschlauchsatz kostengünstig und muss während des Verfahrens nicht geändert werden. Zudem kann das Verfahren komplett an der Behandlungsmaschine durchgeführt werden und noch vor der Konnektion des Patienten vollständig abgeschlossen werden, so dass der Patient in der Regel während der Vorbereitung der Behandlungsmaschine abwesend sein kann.

Die Schnittstellen für den Blutschlauchsatz sind dabei der Substituat-Port, über welchen Substituat in die Substituatleitung eingeführt wird, sowie der Rinse-Port, über welchen benutztes Substituat wieder abgeführt werden kann. Sowohl Substituat-Port als auch Rinse-Port verfügen üblicherweise über Ventile, mit welchen sie zur Anwendung des Verfahrens geschlossen und geöffnet werden können.

Vorteilhafterweise bilden bei dem erfindungsgemäßen Verfahren das Füllen und das Spülen einen kontinuierlichen Vorgang. Hierdurch ergibt sich insbesondere keinerlei Unterbrechung des Flüssigkeitsstroms, womit die sonst gegebene Gefahr, dass durch zeitweiliges Unterbrechen des nachströmenden Flüssigkeitsstroms in Folge von Luftblasenförderung eine Schaumbildung auftritt, vermieden wird. Hierbei werden also nicht nur arterielle und venöse Leitungen gleichzeitig gefüllt bzw. gespült, sondern dieser Füll- und Spülvorgang erfolgt auch kontinuierlich. Neben der eben erwähnten Erhöhung der Sicherheit erlaubt auch dies eine einfachere Automatisierung, da insbesondere auf den Einsatz von Ventilen am Blutschlauchsatz verzichtet werden kann.

Weiterhin vorteilhafterweise erfolgt dabei das Füllen und das Spülen durch den gleichen Vorgang. Hierdurch muss nicht extra zwischen Füll- und Spülvorgang umgeschaltet werden, so dass sich der Steuerungs- bzw. Bedienaufwand für das Verfahren vermindert und die Sicherheit erhöht.

Vorteilhafterweise erfolgt das gleichzeitige Füllen und/oder Spülen der arteriellen und der venösen Leitung dadurch, dass die Blutpumpe im Blutschlauchsatz Substituat befördert, während gleichzeitig Substituat aus der Substituatleitung zugeführt wird. So muss lediglich auf bereits vorhandene Komponenten des Blutschlauchsatzes zurückgegriffen werden, um den Füll- und/oder Spülvorgang zu steuern. Das Substituat aus der Substituatleitung wird dabei in den Blutschlauchsatz zugeführt, während die Blutpumpe dafür sorgt, dass sowohl die arterielle als auch die venöse Leitung mit dem Substituat durchströmt werden. Hierdurch kann auf Ventile verzichtet werden und sowohl die arterielle als auch die venöse Leitung gleichzeitig gefüllt bzw. gespült werden.

Vorteilhafterweise fördert während des Füll- und/oder Spülvorgangs die Blutpumpe eine Menge n an Substituat, wobei der saugseitigen Leitung zur Blutpumpe eine Menge m+n an Substituat aus der Substituatleitung zugeführt wird. Hierdurch pumpt die Blutpumpe einen Teil n des aus der Substituatleitung zugeführten Substituats durch den druckseitigen Teil des Blutschlauchsatzes, während der saugseitige Teil des Blutschlauchsatzes ab der Zuleitung der Substituatleitung von der verbleibenden Menge m des Substituats aus der Substituatleitung direkt durchströmt wird. Bei diesem Verfahren werden also beide Teile des Blutschlauchsatzes gleichzeitig parallel und in gleicher Richtung durchströmt, wobei beide Substituatströme nach dem Füllen des Blutschlauchsatzes am Rinse-Port wieder zusammentreffen und von dort abgeleitet werden.

Auf diese Art und Weise ergibt sich ein einfaches Verfahren, mit welchem der Blutschlauchsatz sicher und schnell befüllt und gespült werden kann, wobei lediglich die Mengen gesteuert werden müssen, welche von der Blutpumpe gefördert werden bzw. welche aus der Substituatleitung zugeführt werden.

Vorteilhafterweise läuft dabei die Blutpumpe rückwärts. Insbesondere wenn das Pumpsegment für die Blutpumpe in der arteriellen Leitung integriert ist, pumpt die Blutpumpe damit direkt Substituat durch die arterielle Leitung zum Rinse-Port, während das restliche Substituat den Dialysator und die venöse Leitung durchströmt und sich dann im Rinse-Port mit dem direkt von der Blutpumpe gepumpten Substituat vereinigt.

Alternativ kann während des gleichzeitigen Füllens und/oder Spülens der arteriellen und der venösen Leitung die Blutpumpe zumindest einen Teil des Substituats im Kreislauf aus arterieller Leitung, Dialysator und venöser Leitung zirkulieren, während zusätzlich Substituat aus der Substituatleitung zugeführt wird. Das Substituat fließt also nicht mehr parallel durch die arterielle und die venöse Leitung, sondern zirkuliert durch diese, während das am Anschluss der Substituatleitung in den Blutschlauchsatz zugegebene Substituat am Rinse-Port wieder abfließt. Auch dies ermöglicht ein einfaches und sicheres Verfahren zum Füllen und Spülen des Blutschlauchsatzes. Insbesondere ist es bei diesem Verfahren nicht entscheidend, wieviel Substituat aus der Substituatleitung zugeführt wird. Die Blutpumpe saugt an der Verbindungsstelle zwischen arterieller und venöser Leitung Substituat der Menge n in den extrakorporalen Blutkreislauf und zirkuliert dort das Substituat, so dass sich eine besonders effektive Spülung ergibt. Frisches Substituat der Menge m fließt dabei über die Substituatleitung zu und verdünnt damit immer wieder das gebrauchte Substituat, so dass im Bereich nach der Zuleitung der Substituatleitung Substituat der Menge m+n fließt, welches teilweise, und zwar mit der Menge m, über den Rinse-Port wieder abfließt. Insbesondere kann dabei über die Substituatleitung weniger Substituat zugeführt werden, als von der Blutpumpe im extrakorporalen Blutkreislauf zirkuliert wird, so dass sich eine besonders effektive Füllung und Spülung ergibt. Vorteilhafterweise liegt die Zugabestelle für die Menge m an Substituat dabei an der druckseitigen Leitung der vorwärts laufenden Blutpumpe.

Erfindungsgemäß ist bei dem erfindungsgemäßen Verfahren das Pumpsegment für die Blutpumpe in der arteriellen Leitung angeordnet. Weiterhin wird das Substituat zwischen Blutpumpe und Dialysator zugeführt, am Prädilutionszugang des Blutschlauchsatzes. So durchströmt frisch zugegebenes Substituat zumindest teilweise zuerst den Dialysator, wodurch dieser besonders effektiv gereinigt wird.

Läuft die Blutpumpe rückwärts, wird dadurch die Menge n an Substituat, welche von der Blutpumpe gepumpt wird, durch die arterielle Leitung in Richtung Rinse-Port gefördert. Die Menge m + n an Substituat, welche bei diesem Verfahren aus der Substituatleitung in den Prädilutionszugang des Blutschlauchsatzes zugeführt wird, wird also teilweise von der Blutpumpe in die arterielle Leitung gepumpt, während der Rest durch den Dialysator und die venöse Leitung strömt.

Bei der zweiten Verfahrensalternative läuft die Blutpumpe dagegen vorwärts und saugt Substituat an der Verbindung zwischen venöser und arterieller Leitung aus der venösen Leitung in die arterielle Leitung und pumpt dieses weiter durch den Dialysator und wieder in die venöse Leitung. Das Substituat wird dabei über den Prädilutionszugang zugeführt und mischt sich mit dem bereits zirkulierenden Substituat. In dem gleichen Maße, wie das Substituat am Prädilutionszugang zugeführt wird, wird es am Rinse-Port wieder abgeführt, sobald der Blutschlauchsatz gefüllt ist.

Zwar kann das erfindungsgemäße Verfahren auch unter Verwendung von Beuteln zum Füllen und Spülen und entsprechenden Beuteln zum Auffangen der benutzten Lösung betrieben werden, besonders vorteilhafterweise kommt es aber in einem System mit integrierter Substituataufbereitung zum Einsatz. Der Substituat-Port und der Rinse-Port sind dabei Teil dieses Systems, wobei über den Substituat-Port das frische Substituat bereit gestellt wird, während über den Rinse-Port das benutzte Substituat zur Aufbereitung zurückgeführt wird. Dies bedeutet insbesondere, dass der Blutschlauchsatz in einen Kreislauf integriert ist.

Weiterhin vorteilhafterweise verfügen dabei beide Ports über ein maschinenseitiges Ventil, so dass die Ventile nicht Bestandteil des Blutschlauchsatzes sein müssen. Insbesondere muss bei einem solchen System mit integrierter Substituataufbereitung nicht mehr auf Beutel zurückgegriffen werden, so dass die Kosten für die Beschaffung, Lagerhaltung, Anwendung und Entsorgung dieser Beutel entfallen. Ein besonderer Vorteil des erfindungsgemäßen Verfahrens besteht insbesondere darin, dass bestehende Systeme mit integrierter Substituataufbereitung nicht hardwaremäßig verändert werden müssen, um das erfindungsgemäße Verfahren anzuwenden. Es bedarf dazu lediglich einer neuen Softwareansteuerung, so dass bestehende Systeme auf einfache Art und Weise mit dem neuen Verfahren ausgestattet werden können.

Vorteilhafterweise erfolgt dabei bei dem erfindungsgemäßen Verfahren die Ansteuerung des Rinse-Ports und der Blutpumpe automatisch. Es müssen bei dem erfindungsgemäßen Verfahren lediglich die Verbindungen des Schlauchsatzes am Anfang hergestellt werden, während der gesamte Füll- und Spülvorgang automatisch erfolgt. Hierdurch wird der Dialyseschwester sehr viel Arbeit erspart, wobei gleichzeitig Fehlermöglichkeiten durch falsche Bedienung ausgeschlossen werden.

Erfindungsgemäß wird das Substituat über eine Substituatpumpe zugeführt. Dies ermöglicht eine genaue Steuerung der zugegebenen Menge, so dass das Füllen und das Spülen äußerst kontrolliert erfolgen kann.

Vorteilhafterweise erfolgt die Ansteuerung der Substituatpumpe dabei automatisch, so dass auch hier keine manuellen Bedienschritte von Nöten sind, so dass damit verbundene Fehlerquellen ausgeschlossen sind und Zeit gespart werden kann.

Weiterhin vorteilhafterweise ist ein Pumpsegment für die Substituatpumpe dabei in der Substituatleitung integriert. Insbesondere bei einem Anschluß an ein System mit integrierter Substituataufbereitung muss dieses System hierdurch hardwaremäßig nicht verändert werden, da alle zum Betreiben des Verfahren notwendigen Komponenten außerhalb des Systems zur Verfügung stehen.

Vorteilhafterweise erfolgt auch die Ansteuerung der Substituatpumpe automatisch. Hierzu sollte vorteilhafterweise das System mit integrierter Substituataufbereitung mit einer neuen Software ausgestattet werden, welche das erfindungsgemäße Zusammenwirken von Substituat-Port, Rinse-Port, Blutpumpe und Substituatpumpe steuert. Weiterer insbesondere hardwareseitiger Veränderungen bedarf es nicht, so dass bestehende Systeme einfach mit dem neuen Verfahren ausgestattet werden können.

Vorteilhafterweise kommt dabei zum Verbinden der arteriellen Leitung, der venösen Leitung und des Rinse-Port ein T-Konnektor zum Einsatz, so dass diese Verbindung auf einfache und sichere Weise hergestellt werden kann.

Während beim gleichzeitigen Spülen der arteriellen und der venösen Leitung das Substituat über den Rinse-Port abgeführt wird, ist es ebenso möglich, während dem Spülen bzw. dem Zirkulieren des Substituats auch über den Dialysator Substituat abzuführen.

Weiterhin umfasst die vorliegende Erfindung eine Blutbehandlungsmaschine gemäß Anspruch 15.

Weiterhin umfasst die vorliegende Erfindung ein extrakorporales Blutbehandlungssystem aus einer solchen Blutbehandlungsmaschine und einem Blutschlauchsatz, der ein Pumpsegment für eine Blutpumpe, eine mit einem Eingang eines Dialysators verbindbare arterielle Leitung und eine mit einem Ausgang eines Dialysators verbindbare venöse Leitung umfasst, sowie eine Substituatleitung und einen Konnektor, wobei der Konnektor mit der arteriellen Leitung, der venösen Leitung und einem Rinse-Port verbindbar ist, wobei ein Pumpsegment für die Substituatpumpe in der Substituatleitung integriert ist. Durch die Integration des Pumpsegments für die Substituatpumpe in der Substituatleitung ist insbesondere der Anschluss an ein System mit integrierter Substituataufbereitung möglich, ohne dieses hardwaremäßig zu verändern. So lässt sich mit dem erfindungsgemäßen Blutschlauchsatz das erfindungsgemäße Verfahren zur Füllung und Spülung des Blutschlauchsatzes besonders einfach und kostengünstig umsetzen.

Ausführungsbeispiele der vorliegenden Erfindung werden nun anhand von Zeichnungen näher beschrieben. Dabei zeigen:
- Figur 1:: ein erstes Ausführungsbeispiel des erfindungsgemäßen Füll- und Spülvorgangs,
- Figur 2:: ein erstes Ausführungsbeispiel des erfindungsgemäßen Zirkulierens und
- Figur 3:: ein zweites Ausführungsbeispiel des erfindungsgemäßen Füll- und Spülvorgangs.

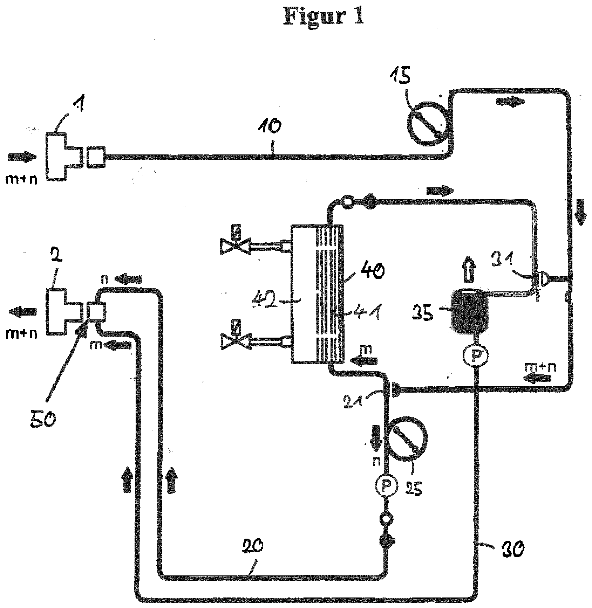

In Figur 1 ist ein erstes Ausführungsbeispiel der Erfindung gezeigt. Das extrakorporale Blutbehandlungssystem mit integrierter Substituataufbereitung umfasst dabei maschinenseitig den Substituat-Port 1 und den Rinse-Port 2. Über den Substituat-Port 1 wird frisches Substituat in den Blutschlauchsatz abgegeben, während über den Rinse-Port 2 gebrauchtes Substituat wieder abgeführt und aufbereitet wird.

Der eigentliche Blutschlauchsatz besteht aus einer arteriellen Leitung 20 und einer venösen Leitung 30. In der arteriellen Leitung 20 befindet sich das Pumpsegment für die Blutpumpe 25, in der venösen Leitung 30 der Luftabscheider 35. Der Dialysator 40 umfasst zwei Kammern, eine Dialysatkammer 42 für die Spülflüssigkeit und eine Blutkammer 41, über welche der Dialysator 40 in den extrakorporalen Blutkreislauf integriert wird. Hierzu ist die arterielle Leitung 20 hinter der Blutpumpe 25 mit dem Eingang der Blutkammer 41 des Dialysators 40 verbunden. Der Ausgang der Blutkammer 41 ist mit der venösen Leitung 30 verbunden, wobei aus der Blutkammer 41 strömende Flüssigkeit zuerst in den Luftabscheider 35 gelangt, um eventuell auftretende Luftblasen abzuscheiden. Zwischen der Blutpumpe 25 und dem Eingang der Blutkammer 41 befindet sich in der arteriellen Leitung 20 der Prädilutionszugang 21. Zwischen dem Ausgang der Blutkammer 41 und dem Luftabscheider 35 befindet sich in der venösen Leitung 30 der Postdilutionszugang, der jedoch bei den vorliegenden Ausführungsbeispielen des erfindungsgemäßen Verfahrens nicht zum Einsatz kommt. Die Substituatleitung 10 ist am Prädilutionszugang angeschlossen und verfügt über ein Pumpsegment für eine Substituatpumpe 15.

Zur Durchführung des erfindungsgemäßen Verfahrens zur Füllung und Spülung des Blutschlauchsatzes ist die Substituatleitung 10 am Substituat-Port 1 des Blutbehandlungssystems angeschlossen. Die patientenseitigen Enden der venösen Leitung 30 und der arteriellen Leitung 20 sind über einen T-Konnektor 50 kurzgeschlossen und mit dem Rinse-Port 2 des Blutbehandlungssystems verbunden. Sowohl Substituat-Port 1 als auch Rinse-Port 2 sind geöffnet.

Bei der in Figur 1 gezeigten Verfahrensvariante wird eine Menge m + n Substituat mittels der Substituatpumpe 15 über den Substituat-Port 1 angesaugt und über den Prädilutionszugang 21 in die arterielle Leitung 20 zwischen Blutpumpe 25 und Eingang des Dialysators 40 eingespeist. Die Blutpumpe 25 läuft in umgekehrter Drehrichtung und pumpt eine Menge n an Substituat direkt durch die arterielle Leitung 20 zum T-Konnektor 50 und damit in den Rinse-Port 2. Der verbleibende Teil des Substituats, also die Menge m, durchströmt zwangsläufig die Blutkammer 41 des Dialysators 40 und danach den Luftabscheider 35, bevor dieser Teilstrom durch die venöse Leitung 30 ebenfalls in den T-Konnektor 50 und damit in den Rinse-Port 2 strömt.

Bei diesem Ausführungsbeispiel werden die arterielle Leitung und die venöse Leitung beim Spülen also parallel und gleichzeitig von zwei Teilströmen durchströmt. Die Teilströme trennen sich beim Prädilutionszugang 21 und vereinigen sich wieder im Rinse-Port, von wo aus sie der Substituataufbereitung des Blutbehandlungssystems zugeführt werden. Auf diese Weise können sowohl venöse als auch arterielle Leitungen gleichzeitig gefüllt werden, wobei zur Steuerung dieses Vorgangs lediglich die Blutpumpe 25 und die Substituatpumpe 15 angesteuert werden müssen, ohne dass am Blutschlauchsatz weitere Ventile vorgesehen sein müssen. Auch geschehen so Füll- und Spülvorgang in einem einzigen Arbeitsgang, da das Substituat aus dem Substituat-Port 1 zuerst den Blutschlauchsatz füllt und dann bei weiter laufenden Pumpen durchspült, wobei das gebrauchte Substituat über den geöffneten Rinse-Port 2 abfließt.

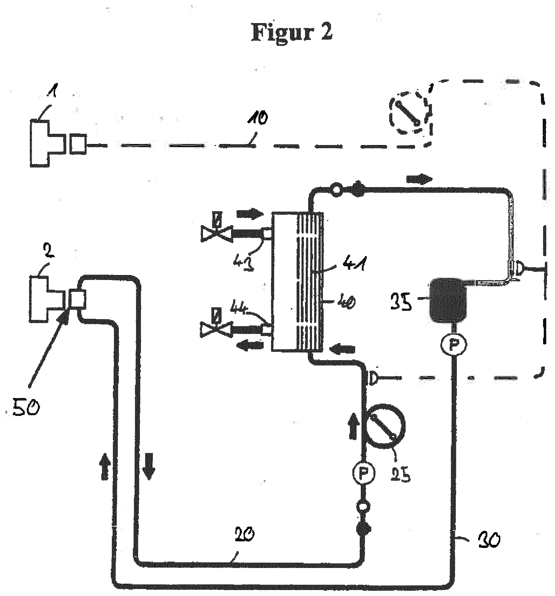

In Figur 2 wurden nun sowohl Substituat-Port 1 als auch Rinse-Port 2 geschlossen, so dass kein Substituat mehr in das Blutschlauchsystem zufließt und auch kein Substituat mehr abfließt. In diesem Vorzirkulierbetrieb arbeitet die Blutpumpe 25 nun in ihrer üblichen Drehrichtung und fördert somit vorwärts. Das Substituat zirkuliert im Blutschlauchsatz nun im Kreislauf in der Reihenfolge Dialysator 40, Luftabscheider 35, venöse Patientenleitung 30, T-Konnektor 50, arterielle Patientenleitung 20, bevor es wieder in die Blutpumpe 25 gelangt und von dort aus weiter gepumpt wird. Damit ist auch der Übergang vom Spülbetrieb in den Vorzirkulierbetrieb möglich, ohne dass Anschlüsse geändert werden müssen oder Ventile am Blutschlauchsatz geöffnet oder geschlossen werden müssen. Wiederum ist also die Steuerung allein über das Öffnen und Schließen der maschinenseitigen Ports und die Ansteuerung der Pumpen möglich.

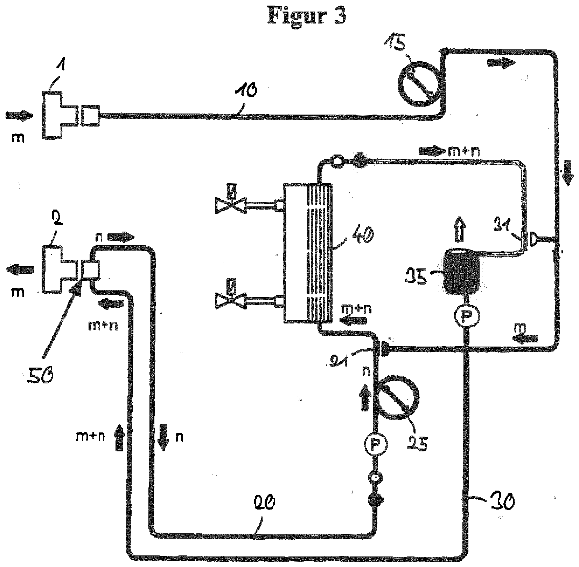

Figur 3 zeigt nun ein zweites Ausführungsbeispiel des erfindungsgemäßen Verfahrens zur Füllung und Spülung des Blutschlauchsatzes. Der Aufbau und der Anschluss des Blutschlauchsatzes ist identisch zum ersten Ausführungsbeispiel, das Füll- und Spülverfahren erfolgt nun aber bei im Blutschlauchsatz zirkulierender Flüssigkeitsströmung.

Hierbei wird bei laufender Substituatpumpe 15 permanent eine Menge m an Substituat in den Prädilutionszugang 21 des Blutschlauchsatzes gepumpt. Über den T-Konnektor wird permanent die gleiche Menge m gebrauchtes Substituat über den Rinse-Port abgegeben, während die Blutpumpe 25 in der arteriellen Leitung 20 in Vorwärtsrichtung arbeitet und eine Menge n an Substituat pumpt. Im Prädilutionszugang 21 vereinigen sich also die Substituatströme aus der Blutpumpe 25 und aus der Substituatleitung 10 zu einem Substituatstrom der Menge m + n, welcher über die Blutkammer 41 des Dialysators 40 weiter in den Luftabscheider 35 und von dort aus über die venöse Leitung 30 zum T-Konnektor 50 fließt. Beim T-Konnektor 50 fließt nun eine Menge m an gebrauchtem Substituat über den Rinse-Port ab, während der Rest des Flüssigkeitsstroms der Menge n von der Blutpumpe 25 in die arterielle Leitung 20 gesogen wird. Bei dieser Verfahrensvariante werden also die arterielle Leitung 20 und die venöse Leitung 30 gleichzeitig in Reihe durchströmt. Das Substituat durchströmt dabei den Blutschlauchsatz in der Reihenfolge Dialysator 40, Luftabscheider 35, venöse Patientenleitung 30, T-Konnektor 50, arterielle Patientenleitung 20, um dann wieder zur Blutpumpe 25 zu gelangen und von dort aus weiter gepumpt zu werden.

Um zum Vorzirkulieren überzugehen, muss lediglich die Substituatpumpe 15 abgeschaltet werden und der Substituat-Port 1 und der Rinse-Port 2 geschlossen werden. Die Blutpumpe 25 läuft einfach weiter und zirkuliert das Substituat wie bereits in Figur 2 gezeigt im Blutschlauchsatz. Durch dieses Verfahren geschieht nicht nur das Füllen und das Spülen des Blutschlauchsatzes in einem Vorgang, sondern es erfolgt auch das Spülen und das danach folgende Vorzirkulieren übergangslos.

Zum Beenden des Vorzirkulierens wird die Blutpumpe 25 angehalten. Dann werden die arterielle Leitung 20 sowie die venöse Leitung 30 vom T-Konnektor 50 getrennt, so dass der Patient an diese Leitungen konnektiert werden kann.

Die Änderungen zum Einsatz des erfindungsgemäßen Verfahrens zur Füllung und Spülung des Blutschlauchsatzes, welche an bestehenden Blutbehandlungssystemen vorgenommen werden müssen, beschränken sich dabei auf den Einsatz des speziellen T-Konnektors 50, der als kostengünstiger Einmalartikel ausgeführt werden kann, und der integrierten Substituatpumpe, sowie die Änderung des Steuerprogramms der Behandlungsmaschine, wobei keine neuen Komponenten berücksichtigt bzw. angesteuert werden müssen. Die Änderungen beschränken sich deshalb auf Änderungen der Software. Insbesondere ist die Notwendigkeit einer zusätzlichen, störenden und mit Kosten verbundenen elektrischen Verkabelung nicht gegeben.

Weiterhin ergibt sich der Vorteil, dass der Blutschlauchsatz komplett maschinengesteuert und kontrolliert befüllt und gespült wird und zudem ohne Unterbrechung zum Vorzirkulieren des Substituats übergegangen wird. Insbesondere erfolgt die Füllung und Spülung dabei kontinuierlich und ohne jede Unterbrechung des Flüssigkeitsstroms, was die Sicherheit zusätzlich erhöht. Die mit manuellen Behandlungsschritten verbundenen Fehlerquellen sind damit ausgeschlossen.

## Patentansprüche

1. Verfahren zur Füllung und Spülung eines Blutschlauchsatzes, der ein Pumpsegment für eine Blutpumpe, eine mit einem Eingang eines Dialysators verbundene arterielle Leitung, eine mit einem Ausgang eines Dialysators verbundene venöse Leitung sowie einen Prädilutionszugang, welcher sich zwischen der Blutpumpe und dem Eingang des Dialysators in der arteriellen Leitung befindet, umfasst, über eine Substituatleitung, wobei das Verfahren die Schritte umfasst:
- Verbinden der arteriellen Leitung mit der venösen Leitung sowie Verbinden dieser beiden Leitungen mit einem Rinse-Port,
- Verbinden der Substituatleitung mit einem Substituat-Port,
- Öffnen des Rinse-Ports
- Füllen der arteriellen und der venösen Leitung,
- Spülen der arteriellen und der venösen Leitung,
- Schließen des Rinse-Ports,
- Zirkulieren des Substituats im Kreislauf aus arterieller Leitung, Dialysator und venöser Leitung durch die Blutpumpe,
wobei die Blutpumpe in der arteriellen Leitung angeordnet ist und das Füllen und/oder das Spülen der arteriellen und der venösen Leitung gleichzeitig erfolgt, wobei Substituat aus der Substituatleitung zugeführt wird,
wobei am Prädilutionszugang des Blutschlauchsatzes über eine Substituatpumpe Substituat zwischen Blutpumpe (25) und Dialysator (40) zugeführt wird.

2. Verfahren nach Anspruch 1, wobei das Füllen und das Spülen einen kontinuierlichen Vorgang darstellen.

3. Verfahren nach Anspruch 1, wobei das Füllen und das Spülen durch den gleichen Vorgang erfolgen.

4. Verfahren nach einem der vorhergehenden Ansprüche, wobei das gleichzeitige Füllen und/oder Spülen der arteriellen und der venösen Leitung dadurch erfolgt, dass die Blutpumpe im Blutschlauchsatz Substituat befördert, während gleichzeitig Substituat aus der Substituatleitung zugeführt wird.

5. Verfahren nach Anspruch 4, wobei die Blutpumpe während des Füll- und/oder Spülvorgangs eine Menge n an Substituat befördert und der saugseitigen Leitung der Blutpumpe eine Menge m + n an Substituat aus der Substituatleitung zugeführt wird.

6. Verfahren nach Anspruch 4 oder 5, wobei die Blutpumpe rückwärts läuft.

7. Verfahren nach Anspruch 4, wobei die Blutpumpe zumindest einen Teil des Substituats im Kreislauf aus arterieller Leitung, Dialysator und venöser Leitung zirkuliert, während zusätzlich Substituat aus der Substituatleitung zugeführt wird.

8. Verfahren nach Anspruch 1, wobei Substituat-Port und Rinse-Port Teil eines Systems mit integrierter Substituataufbereitung sind.

9. Verfahren nach Anspruch 1, wobei die Ansteuerung des Rinse-Ports und der Blutpumpe automatisch erfolgt.

10. Verfahren nach Anspruch 1, wobei ein Pumpsegment für eine Substituatpumpe in der Substituatleitung integriert ist.

11. Verfahren nach Anspruch 1, wobei die Ansteuerung der Substituatpumpe automatisch erfolgt.

12. Verfahren nach Anspruch 1, wobei die arterielle Leitung, die venöse Leitung und der Rinse-Port über einen T-Konnektor verbunden werden.

13. Verfahren nach Anspruch 1, wobei beim gleichzeitigen Spülen der arteriellen und der venösen Leitung Substituat über den Rinse-Port abgeführt wird.

14. Verfahren nach Anspruch 1, wobei beim gleichzeitigen Spülen der arteriellen und der venösen Leitung oder beim Zirkulieren Substituat über den Dialysator abgeführt wird.

15. Blutbehandlungsmaschine mit integrierter Substituataufbereitung zur Durchführung eines Verfahrens nach einem der vorangegangenen Ansprüche, mit einem Substituat-Port (1) und einem Rinse-Port (2), welche als Schnittstelle für den Blutschlauchsatz dienen und über Ventile verfügen, mit welchen sie zur Anwendung des Verfahrens geschlossen und geöffnet werden können, und mit einer Blutpumpe (25), einer Substituatpumpe und einem Steuerprogramm, wobei das Steuerprogramm das Zusammenwirken von Substituat-Port, Rinse-Port, Blutpumpe und Substituatpumpe automatisch derart steuert, dass die folgenden Schritte ausgeführt werden:
- Öffnen des Rinse-Ports (2)
- Füllen der arteriellen (20) und der venösen Leitung (30),
- Spülen der arteriellen (20) und der venösen Leitung (30),
- Schließen des Rinse-Ports (2),
- Zirkulieren des Substituats im Kreislauf aus arterieller Leitung (20), Dialysator (40) und venöser Leitung (30) durch die Blutpumpe (25),
wobei das Füllen und/oder das Spülen der arteriellen (20) und der venösen Leitung (30) gleichzeitig erfolgt, indem die Blutpumpe (25) im Blutschlauchsatz Substituat befördert, während gleichzeitig über die Substituatpumpe Substituat an einem Prädilutionszugang zwischen Blutpumpe (25) und Dialysator (40) zugeführt wird.

16. Extrakorporales Blutbehandlungssystem aus einer Blutbehandlungsmaschine gemäß Anspruch 15 und einem Blutschlauchsatz, welcher ein Pumpsegment für eine Blutpumpe, eine mit einem Eingang eines Dialysators verbindbare arterielle Leitung, eine mit einem Ausgang eines Dialysators verbindbare venöse Leitung sowie einen Prädilutionszugang, welcher sich zwischen der Blutpumpe und dem Eingang des Dialysators in der arteriellen Leitung befindet, umfasst, sowie eine Substituatleitung und einen Konnektor, wobei der Konnektor mit der arteriellen Leitung, der venösen Leitung und einem Rinse-Port verbindbar ist, wobei ein Pumpsegment für eine Substituatpumpe in der Substituatleitung integriert ist.

## Claims

1. A method for the filling and flushing of a blood tube set that comprises a pump segment for a blood pump, an arterial line connected to an inlet of a dialyzer, a venous line connected to an outlet of a dialyzer, and a predilution port that is located between the blood pump and the inlet of the dialyzer in the arterial line, via a substituate line, the method comprising the steps:
- connecting the arterial line to the venous line and connecting these two lines to a rinse port;
- connecting the substituate line to a substituate port;
- opening the rinse port;
- filling the arterial line and the venous line;
- flushing the arterial line and the venous line;
- closing the rinse port;
- circulating the substituate in the circuit of arterial line, dialyzer and venous line by the blood pump,
wherein the blood pump is arranged in the arterial line and the filling and/or the flushing of the arterial line and the venous line takes/take place simultaneously,
wherein substitute is supplied from the substituate line; and
wherein substituate is supplied at the predilution port of the blood tube set between the blood pump (25) and the dialyzer (40).

2. A method in accordance with claim 1, wherein the filling and the flushing represent a continuous process.

3. A method in accordance with claim 1, wherein the filling and the flushing take place by the same process.

4. A method in accordance with one of the preceding claims, wherein the simultaneous filling and/or flushing of the arterial line and of the venous line takes/take place in that the blood pump transports substituate in the blood tube set, while substituate is simultaneously supplied from the substituate line.

5. A method in accordance with claim 4, wherein the blood pump transports an amount n of substituate during the filling and/or flushing process and an amount m + n of substituate is supplied from the substituate line to the suction-side line of the blood pump.

6. A method in accordance with claim 4 or claim 5, wherein the blood pump runs backwards.

7. A method in accordance with claim 4, wherein the blood pump circulates at least some of the substituate in the circuit of arterial line, dialyzer and venous line while substituate is additionally supplied from the substituate line.

8. A method in accordance with claim 1, wherein the substituate port and the rinse port are part of a system with an integrated substituate line.

9. A method in accordance with claim 1, wherein the control of the rinse port and of the blood pump takes place automatically.

10. A method in accordance with claim 1, wherein a pump segment for a substituate pump is integrated in the substituate line.

11. A method in accordance with claim 1, wherein the control of the substituate pump takes place automatically.

12. A method in accordance with claim 1, wherein the arterial line, the venous line and the rinse port are connected via a T connector.

13. A method in accordance with claim 1, wherein substituate is drained off via the rinse port with the simultaneous flushing of the arterial line and of the venous line.

14. A method in accordance with claim 1, wherein substituate is drained off via the dialyzer with the simultaneous flushing of the arterial line and of the venous line or during circulation.

15. A blood treatment machine with integrated substituate preparation for carrying out a method in accordance with one of the preceding claims, having a substituate port (1) and a rinse port (2) which serve as an interface for the blood tube set and have valves by which they can be opened and closed for the application of the method, und having a blood pump (25), a substituate pump and a control program, with the control program automatically controlling the cooperation of substituate port, rinse port, blood pump and substituate pump such that the following steps are carried out:
- Opening the rinse port (2)
- Filling the arterial line (20) and the venous line (30),
- Flushing the arterial line (20) and the venous line (30),
- Closing the rinse port (2),
- Circulating the substituate in the circuit of arterial line (20), dialyzer (40) and venous line (30) by the blood pump (25),
with the filling and/or flushing of the arterial line (20) and the venous line (30) taking place simultaneously in that the blood pump (25) transports substituate in the blood tube set while substituate is simultaneously supplied via the substituate pump at a predilution port between the blood pump (25) and the dialyzer (40).

16. An extracorporeal blood treatment system of a blood treatment machine in accordance with claim 15 and a blood tube set that comprises a pump segment for a blood pump, an arterial line connectable to an inlet of a dialyzer, a venous line connectable to an outlet of a dialyzer, and a predilution port that is located between the blood pump and the inlet of the dialyzer in the arterial line, and a substituate line and a connector, with the connector being connectable to the arterial line, the venous line and a rinse port, with a pump segment for a substituate pump being integrated in the substituate line.

## Revendications

1. Procédé de remplissage et de rinçage d'un jeu de tuyaux de sang, qui comprend un segment de pompe pour une pompe à sang, une ligne artérielle reliée à une entrée d'un dialyseur et une ligne veineuse reliée à une sortie d'un dialyseur ainsi qu'une arrivée de prédilution, qui se trouve dans la ligne artérielle entre la pompe à sang et l'entrée du dialyseur, par le biais d'une ligne de liquide de substitution, le procédé comprenant les étapes consistant à :
- relier la ligne artérielle à la ligne veineuse ainsi que relier ces deux lignes à un port de rinçage,
- relier la ligne de liquide de substitution à un port de liquide de substitution,
- ouvrir le port de rinçage
- remplir la ligne artérielle et la ligne veineuse,
- rincer la ligne artérielle et la ligne veineuse,
- fermer le port de rinçage,
- faire circuler le liquide de substitution dans le circuit formé par la ligne artérielle, le dialyseur et la ligne veineuse au moyen de la pompe à sang,
dans lequel la pompe à sang est disposée dans la ligne artérielle et le remplissage et/ou le rinçage de la ligne artérielle et la ligne veineuse est/sont effectué(s) simultanément, du liquide de substitution étant alimenté depuis la ligne de liquide de substitution,
dans lequel du liquide de substitution est alimenté entre la pompe à sang (25) et le dialyseur (40) au niveau de l'arrivée de prédilution du jeu de tuyaux de sang, par le biais d'une pompe à liquide de substitution.

2. Procédé selon la revendication 1, dans lequel le remplissage et le rinçage constituent un processus continu.

3. Procédé selon la revendication 1, dans lequel le remplissage et le rinçage sont effectués par le même processus.

4. Procédé selon l'une des revendications précédentes, dans lequel le remplissage et/ou rinçage simultané(s) de la ligne artérielle et la ligne veineuse est/sont effectué(s) du fait que la pompe à sang transporte du liquide de substitution dans le jeu de tuyaux de sang, pendant que du liquide de substitution est alimenté simultanément depuis la ligne de liquide de substitution.

5. Procédé selon la revendication 4, dans lequel la pompe à sang transporte une quantité n de liquide de substitution pendant le processus de remplissage et/ou de rinçage et une quantité m + n de liquide de substitution est alimentée depuis la ligne de liquide de substitution dans la ligne côté aspiration de la pompe à sang.

6. Procédé selon la revendication 4 ou 5, dans lequel la pompe à sang fonctionne en arrière.

7. Procédé selon la revendication 4, dans lequel la pompe à sang fait circuler au moins une partie du liquide de substitution dans le circuit formé par la ligne artérielle, le dialyseur et la ligne veineuse, pendant que du liquide de substitution est en plus alimenté depuis la ligne de substitution.

8. Procédé selon la revendication 1, dans lequel le port de liquide de substitution et le port de rinçage font partie d'un système avec traitement intégré du liquide de substitution.

9. Procédé selon la revendication 1, dans lequel la commande du port de rinçage et de la pompe à sang est effectuée automatiquement.

10. Procédé selon la revendication 1, dans lequel un segment de pompe pour une pompe à liquide de substitution est intégré dans la ligne de liquide de substitution.

11. Procédé selon la revendication 1, dans lequel la commande de la pompe à liquide de substitution est effectuée automatiquement.

12. Procédé selon la revendication 1, dans lequel la ligne artérielle, la ligne veineuse et le port de rinçage sont reliés par le biais d'un connecteur en T.

13. Procédé selon la revendication 1, dans lequel, lors du rinçage simultané de la ligne artérielle et la ligne veineuse, du liquide de substitution est évacué par le biais du port de rinçage.

14. Procédé selon la revendication 1, dans lequel, lors du rinçage simultané de la ligne artérielle et la ligne veineuse ou lors de la circulation, du liquide de substitution est évacué par le biais du dialyseur.

15. Machine de traitement du sang avec traitement de liquide de substitution intégré, destinée à exécuter un procédé selon l'une des revendications précédentes, comprenant un port de liquide de substitution (1) et un port de rinçage (2), qui servent d'interface pour le jeu de tuyaux de sang et qui disposent de vannes, au moyen desquelles ils peuvent être fermés et ouverts pour l'application du procédé, et comprenant une pompe à sang (25), une pompe à liquide de substitution et un programme de commande, le programme de commande commandant automatiquement l'action conjointe du port de liquide de substitution, du port de rinçage, de la pompe à sang et de la pompe à liquide de substitution de telle manière que les étapes suivantes sont exécutées :
- ouverture du port de rinçage (2)
- remplissage de la ligne artérielle (20) et la ligne veineuse (30),
- rinçage de la ligne artérielle (20) et la ligne veineuse (30),
- fermeture du port de rinçage (2),
- circulation du liquide de substitution dans le circuit formé par la ligne artérielle (20), le dialyseur (40) et la ligne veineuse (30) au moyen de la pompe à sang (25),
dans lequel le remplissage et/ou le rinçage de la ligne artérielle (20) et la ligne veineuse (30) est/sont effectué(s) simultanément par le fait que la pompe à sang (25) transporte du liquide de substitution dans le jeu de tuyaux de sang, pendant que du liquide de substitution est alimenté simultanément entre la pompe à sang (25) et le dialyseur (40) au niveau d'une arrivée de prédilution, par le biais de la pompe à liquide de substitution.

16. Système de traitement du sang extracorporel constitué d'une machine de traitement du sang selon la revendication 15 et d'un jeu de tuyaux de sang, qui comprend un segment de pompe pour une pompe à sang, une ligne artérielle pouvant être reliée à une entrée d'un dialyseur, une ligne veineuse pouvant être reliée à une sortie d'un dialyseur ainsi qu'une arrivée de prédilution, qui se trouve dans la ligne artérielle entre la pompe à sang et l'entrée du dialyseur, ainsi qu'une ligne de liquide de substitution et un connecteur, le connecteur pouvant être relié à la ligne artérielle, à la ligne veineuse et au port de rinçage, un segment de pompe pour une pompe à liquide de substitution étant intégré dans la ligne de liquide de substitution.
